# EUROPÄISCHE PATENTANMELDUNG

(11) **EP 2 009 001 A1**
(43) Veröffentlichungstag der Anmeldung: **31.12.2008**
(21) Anmeldenummer: 07011965.6
(22) Anmeldetag: 19.06.2007
(51) Int. Cl.: C07D 213/82, C07D 413/04

(54) **Verfahren zur Herstellung von Dioxazin-Derivaten**

(71) Anmelder: Bayer CropScience AG, 40789 Monheim (DE)
(72) Erfinder: PAZENOK, Dr. Sergiy, 42699 (DE); STELZER, Dr. Uwe, Kansas City, MO 64120 (US)

(57) **Zusammenfassung**

Beschreiben wird ein Verfahren zur Herstellung von Dioxazin-Derivaten der Formel (1) 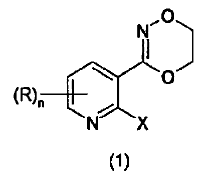 sowie entsprechende Intermediate.

## Beschreibung

Die Erfindung betrifft ein Verfahren zur Herstellung von Dioxazin-Derivaten sowie Intermediate, welche während der Herstellung erhalten werden.

Dioxazine der allgemeinen Formel (1) 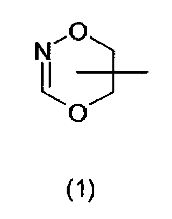 sind wichtige funktionelle Gruppen in einer Vielzahl an organischen Wirkverbindungen. Beispielsweise treten Dioxazinringe in agrochemischen Wirkstoffen (vgl. DE 10 2005 044 108 A1), insbesondere in Dioxazin-Pyridinyl-Suflonylhamstoffen (vgl. US 5,476,936) auf. Darüber hinaus enthalten viele organische Pigmente Dioxazinringe (vgl. DE 10 2005 063 360 A1).

Die Synthese von Dioxazinderivaten verläuft im Allgemeinen über die Umsetzung von entsprechenden Carbonsäureestern mit Hydroxylamin und anschließender Umsetzung mit Dibromethan. Diese Umsetzungskaskade ist in nachfolgender Reaktionsgleichung beispielsweise für Nicotinsäureester gemäß US 5,476,936 verdeutlicht: 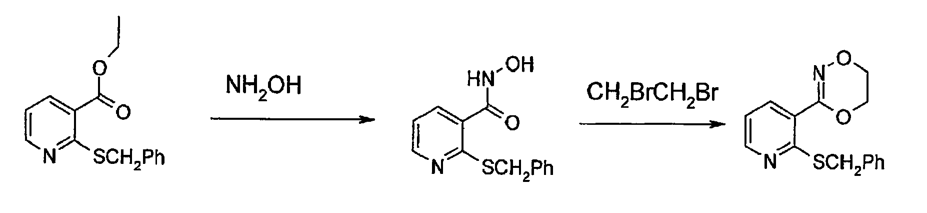 Die geringe Ausbeute von 21 % für die oben beschriebene Reaktion und die Anwendung des hochtoxischen und umweltschädigenden Dibromethans macht die Realisierung eines solchen Verfahrens unattraktiv und teuer.

Es besteht daher Bedarf nach einem kostengünstigen und umweltschonenden Verfahren zur Herstellung von Dioxazin-Derivaten, welches die gewünschten Verbindungen mit guter Ausbeute und hoher Reinheit liefert.

Die Aufgabe der vorliegenden Erfindung ist somit, ein Verfahren zur Herstellung von Dioxazin-Derivaten zur Verfügung zu stellen, welches vorzugsweise mit guten Ausbeuten verläuft und in welchem vorzugsweise auf die Verwendung von hochtoxischen und umweltschädigenden Substanzen, insbesondere von Dibromethan, verzichtet werden kann. Die gewünschten Zielverbindungen sollten dabei vorzugsweise kostengünstig und mit hoher Reinheit erhalten werden.

Die zuvor beschriebene Aufgabe wird erfindungsgemäß gelöst durch ein Verfahren zur Herstellung von Dioxazin-Derivaten der Formel (1) 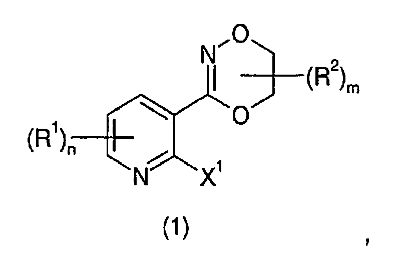 worin die einzelnen Substituenten die folgende Bedeutung aufweisen:
- X¹: Fluor, Chlor, Brom, Iod, SCN, oder S-R³, wobei
R³ für Wasserstoff; gegebenenfalls substituiertes C₁-C₆-Alkyl; gegebenenfalls substituiertes C₃-C₆-Cycloalkyl; -(CH₂)ᵣ-C₆H₅ mit r = ganzzahlige Zahl von 0 bis 6, wobei der Alkylrest -(CH₂)ᵣ- gegebenenfalls substituiert sein kann; oder 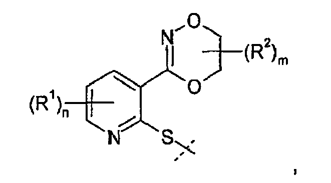 wobei die Substituenten R¹ und R² sowie die Indizes n und m dieselbe Bedeutung haben wie in der allgemeinen Formel (1);
- R¹: Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl-und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
- n: eine ganzzahlige Zahl von 0 bis 2;
- R²: unabhängig voneinander jeweils gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, wobei die Substituenten unabhängig voneinander ausgewählt sein können aus Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, (C₁-C₆-Alkoxy)carbonyl, (C₁-C₆-Alkyl)carbonyl oder C₃-C₆-Trialkylsilyl; und m eine ganzzahlige Zahl von 0 bis 4.

Das erfindungsgemäße Verfahren ist dadurch gekennzeichnet, dass die Dioxazin-Derivate der allgemeinen Formel (1) durch einen Ringschluss ausgehend von einer Verbindung der allgemeinen Formel (2) hergestellt werden (Verfahrensschritt (1)): 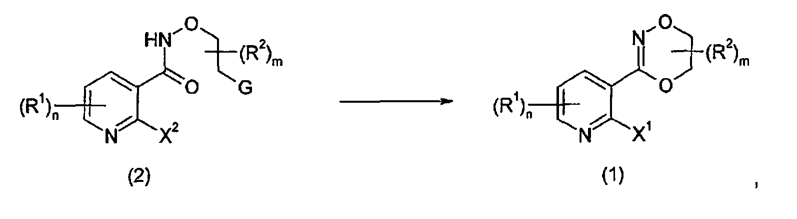 wobei die Substituenten R¹, R² und X¹ sowie die Indizes n und m wie oben stehend definiert sind, G eine Abgangsgruppe darstellt, welche ausgewählt ist aus der Gruppe, bestehend aus Fluor, Chlor, Brom, Iod, -OSO₂-CH₃, -O-SO₂CF₃, -O-SO₂-Ph und -O-SO₂-C₆H₄-Me und X² für Fluor, Chlor, Brom, Iod, SCN, oder S-R³ steht, wobei R³ für Wasserstoff; gegebenenfalls substituiertes C₁-C₆-Alkyl; gegebenenfalls substituiertes C₃-C₆-Cycoalkyl; -(CH₂)ᵣ-C₆H₅ mit r = 0 bis 6, wobei der Alkylrest - (CH₂)ᵣ- gegebenenfalls substituiert sein kann; oder für den Rest 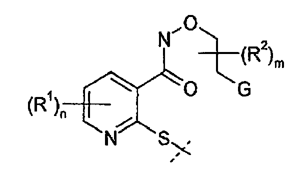 steht, wobei die Substituenten R¹ und R² und die Indizes m und n in dem Rest dieselbe Bedeutung wie in der allgemeinen Formel (1) aufweisen.

Verbindungen der allgemeinen Formel (2) mit G = Cl sind ausgehend von Verbindungen der allgemeinen Formel (3) durch Chlorierung, zum Beispiel mit Thionylchlorid, erhältlich (Verfahrensschritt (2a)):

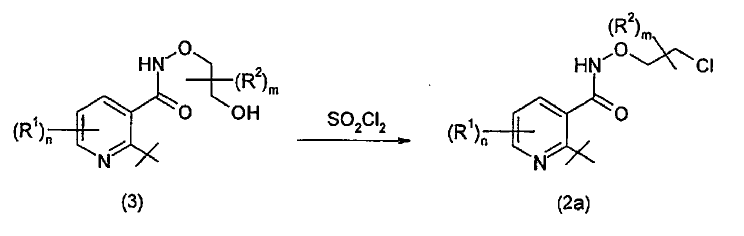

Verbindungen der allgemeinen Formel (2) mit G = Br sind ausgehend von Verbindungen der allgemeinen Formel (3) durch Bromierung erhältlich (Verfahrensschritt (2b)):

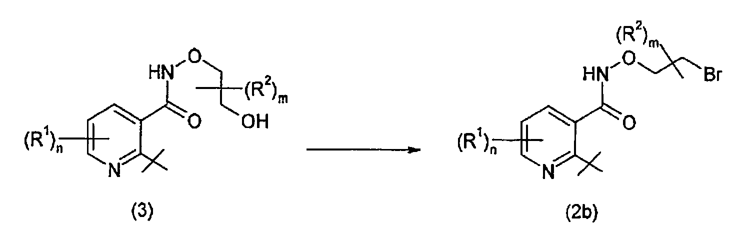

Verbindungen der allgemeinen Formel (2) mit G = -O-SO₂CH₃, G = -O-SO₂-Ph oder G = -O-SO₂-C₆H₄-CH₃ sind ausgehend von Verbindungen der allgemeinen Formel (3) durch Umsetzung mit CH₃SO₂Cl bzw. PhSO₂Cl erhältlich (Verfahrensschritt (2c)):

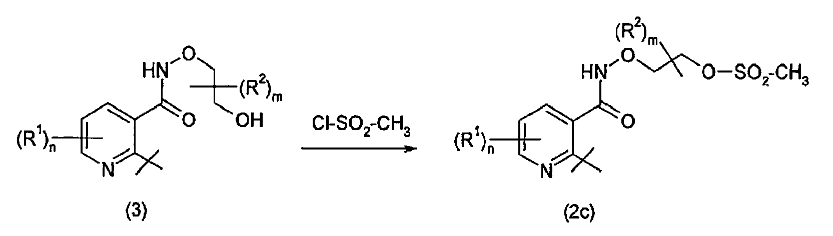

Verbindungen der allgemeinen Formel (2) mit G = -O-SO₂CF₃ sind ausgehend von Verbindungen der allgemeinen Formel (3) durch Umsetzung mit (CF₃SO₂)₂O erhältlich (Verfahrensschritt (2d)):

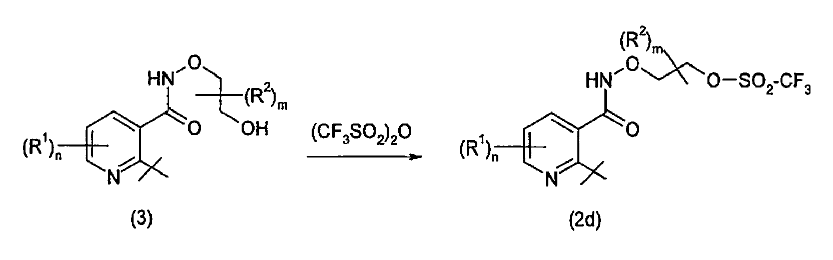

Verbindungen der allgemeinen Formel (2) mit G = F sind ausgehend von Verbindungen der allgemeinen Formel (3) durch Umsetzung mit (CH₃)₂NSF₃, Deoxofluor^{®}, Yarovenko- oder Ishikava-Reagenz erhältlich (Verfahrensschritt (2e)):

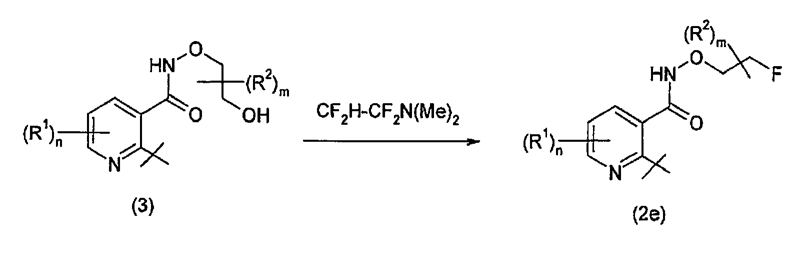

Verbindungen der allgemeinen Formel (2) mit G = I sind ausgehend von Verbindungen der allgemeinen Formel (3) durch Umsetzung mit Phosphor/lod (P/I₂) oder mit CH₃SO₂Cl/KI erhältlich (Verfahrensschritt (2f)):

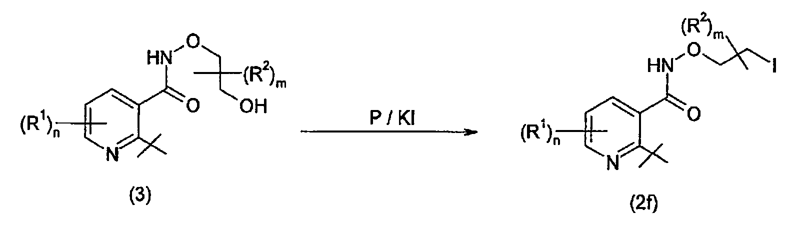

In den oben beschriebenen Verfahren der Verbindungen (2a) bis (2g) sind die Reste in ortho-Position zum Pyridinstickstoff, d.h. die Substituenten zwischen PyridinStickstoff und Amid/Dioxazin-Substituent, nicht näher definiert, wobei im Falle von Dimerstrukturen die Transformation an der Hydroxylgruppe zweimal auftreten kann.

Verbindungen der allgemeinen Formel (3) sind aus dem Stand der Technik bekannt; vgl. hierzu die zeitgleich eingereichte europäische Patentanmeldung der Bayer CropScience AG mit dem Titel "Nicotinamid-Derivate und Verfahren zu deren Herstellung".

In einer Ausführungsform der vorliegenden Erfindung umfasst das erfindungsgemäße Verfahren sowohl die Verfahrensschritte (1) und (2), d.h. das Verfahren wird in dieser Ausführungsform insgesamt durch folgende Reaktionskaskade gekennzeichnet: 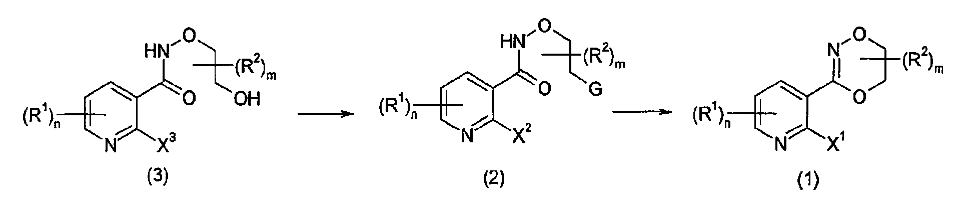 wobei die einzelnen Substituenten die zuvor definierten Bedeutungen aufweisen und X³ für Fluor, Chlor, Brom, Iod, SCN, oder S-R³ steht, wobei R³ für Wasserstoff; gegebenenfalls substituiertes C₁-C₆-Alkyl; gegebenenfalls substituiertes C₃-C₆-Cycoalkyl; -(CH₂)ᵣ-C₆H₅ mit r = 0 bis 6, wobei der Alkylrest -(CH₂)ᵣ- gegebenenfalls substituiert sein kann; oder für den Rest 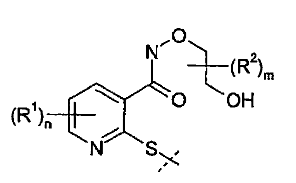 steht, wobei die Substituenten R¹ und R² und die Indizes m und n in dem Rest dieselbe Bedeutung wie in der allgemeinen Formel (1) aufweisen.

Im Rahmen der vorliegenden Erfindung weisen die Substituenten der Verbindungen der allgemeinen Formeln (1) bis (3) vorzugsweise folgende Bedeutung auf:
- X¹, X², X³: Chlor, S-R³, wobei
R³ für gegebenenfalls substituiertes C₁-C₆-Alkyl; gegebenenfalls substituiertes C₃-C₆-Cycoalkyl; -(CH₂)ᵣ-C₆H₅ mit r = 0 bis 4 steht, wobei der Alkylrest -(CH₂)ᵣ- gegebenenfalls substituiert sein kann;
- R¹: Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
- n: 0 oder 1;
- R²: jeweils unabhängig voneinander für gegebenenfalls einfach oder mehrfach, gleich oder verschieden substituiertes C₁-C₄-Alkyl, C₃-C₆-Cycloalkyl, wobei die Substituenten unabhängig voneinander ausgewählt sein können aus Halogen, Cyano, Nitro, Hydroxy, C₁-C₄-Alkoxy, C₁-C₄-Haloalkoxy, C₁-C₄-Alkylthio, C₁-C₄-Alkylsulfinyl und C₁-C₄-Alkylsulfonyl;
- m: eine ganzzahlige Zahl von 0 bis 2; und
- G: Fluor, Chlor, Brom und Iod.

Darüber hinaus sind folgende Bedeutungen der Substituenten der Verbindungen der allgemeinen Formeln (1) bis (3) besonders bevorzugt:
- X¹, X², X³: Chlor, S-R³, wobei
R³ für gegebenenfalls substituiertes C₁-C₆-Alkyl; gegebenenfalls substituiertes C₃-C₆-Cycoalkyl; -(CH₂)ᵣ-C₆H₅ mit r = 0 bis 2 steht, wobei der Alkylrest -(CH₂)ᵣ- gegebenenfalls substituiert sein kann;
- R¹: Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
- n: 0 oder 1;
- R²: gegebenenfalls einfach oder mehrfach, gleich oder verschieden substituiertes C₁-C₄-Alkyl, wobei die Substituenten unabhängig voneinander ausgewählt sein können aus Halogen, Cyano, Nitro, C₁-C₄-Alkoxy, C₁-C₄-Haloalkoxy;
- m: 0 oder 1; und
- G: Chlor.

Darüber hinaus sind folgende Bedeutungen der Substituenten der Verbindungen der allgemeinen Formeln (1) bis (3) insbesondere bevorzugt:
- X¹, X², X³: S-CH₂-C₆H₅;
- n: 0;
- m: 0; und
- G: Chlor.

### Verfahrensschritt (1):

Das erfindungsgemäße Verfahren ist durch den Verfahrensschritt (1) des Ringschlusses der Verbindung der allgemeinen Formel (2) zu der Verbindung der allgemeinen Formel (1) gekennzeichnet.

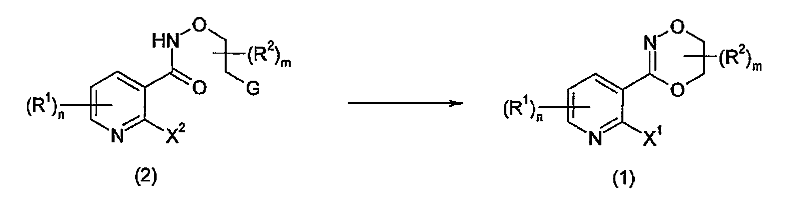

Grundsätzlich bestehen mehrere Möglichkeiten, den Verfahrensschritt (1) durchzuführen:
So ist es in einer ersten Ausgestaltung des Verfahrensschrittes (1) möglich, die Umsetzung unter Ringschluss in Gegenwart von Basen durchzuführen.

Als Basen können in diesem Fall sowohl organische als auch anorganische Basen verwendet werden. Bevorzugt werden anorganische Basen wie beispielsweise LiOH, NaOH, KOH, Ca(OH)₂, Ba(OH)₂, Li₂CO₃, K₂CO₃, Na₂CO₃, NaHCO₃, oder organische Basen wie Amine (beispielsweise bevorzugt Triethylamin, Diethylisopropylamin), Bu₄NOH, Piperidin, Morpholin, Pyridine, Alkylpyridine und DBU verwendet. Besonders bevorzugt werden anorganische Basen wie beispielsweise LiOH, NaOH, KOH, Ca(OH)₂, Ba(OH)₂, Li₂CO₃, K₂CO₃, Na₂CO₃ und NaHCO₃ verwendet. Ganz besonders bevorzugt werden LiOH, NaOH, KOH, K₂CO₃, Na₂CO₃, NaHCO₃ verwendet.

Wenn eine Base in dem Verfahrensschritt (1) des erfindungsgemäßen Verfahrens verwendet wird, so beträgt deren Menge vorzugsweise 0,6 mol bis 4,0 Moläquivalente, besonders bevorzugt 1 bis 3 Moläquivalente, insbesondere 1,2 bis 2,5 Moläquivalente, jeweils bezogen auf die Verbindung der allgemeinen Formel (2).

Der Verfahrensschritt (1) wird im Allgemeinen in Gegenwart eines Lösemittels durchgeführt. Der Verfahrensschritt (1) kann dabei sowohl in Wasser als auch in Gegenwart eines inerten organischen Lösemittels, vorzugsweise eines polaren aprotischen Lösemittels durchgeführt werden. Beispiele organischer Lösemittel, welche im Rahmen der vorliegenden Erfindung verwendet werden können, sind aromatische oder aliphatische Lösemittel wie Benzol, Toluol, Xylol, Mesitylen, Hexan, Heptan, Octan, Cyclohexan; aliphatische und aromatische Halogenwasserstoffe, wie Methylenchlorid, Dichlorethan, Chloroform, Kohlenstofftetrachlorid, Chlorbenzol, Dichlorbenzol; Ether, wie Diethylether, Dibutylether, Diisobutylether, Methyl-tert-butylether, Isopropylethylether, Tetrahydrofuran und Dioxan; weiter auch Dimethylsulfoxid und Säureamidderivate wie N,N-Dimethylformamid, N,N-Dimethylacetamid und N-Methyl-2-pyrollidon; sowie auch Carbonsäureester wie Ethylacetat, oder aber auch Dioxan, Diglym oder Dimethylglycol; Nitrile wie Methylnitril, Butylnitril oder Phenylnitril. Besonders bevorzugt sind Toluol, Xylol, Dichlorbenzol, Chlorbenzol, Ethylacetat, Dichlorethan, N,N-Dimethylformamid, N,N-Dimethylacetamid und Wasser. Insbesondere bevorzugt sind die Lösemittel N,N-Dimethylformamid, N,N-Dimethylacetamid, Ethylacetat, Dichlorethan und Wasser. Allerdings ist die vorliegende Erfindung nicht auf die zuvor beispielhaft genannten Lösemittel beschränkt.

Die Reaktionstemperatur, bei welcher die Ringschlussreaktion gemäß Verfahrensschritt (1) durchgeführt werden kann, kann in weiten Bereichen variieren. Beispielsweise kann die Ringschlussreaktion bei einer Temperatur von 20 bis 100 °C, vorzugsweise 20 bis 70 °C, durchgeführt werden.

Der Verfahrensschritt (1) des erfindungsgemäßen Verfahrens wird im Allgemeinen unter Normaldruck durchgeführt. Es ist jedoch auch möglich, unter erhöhtem Druck oder vermindertem Druck - im Allgemeinen zwischen 0,1 bar und 10 bar - zu arbeiten.

Darüber hinaus ist es möglich, in dem Verfahrensschritt (1) der vorliegenden Erfindung als Edukt für die Ringschlussreaktion eine Verbindung zu verwenden, welche durch die Verfahrensstufe (2), also beispielsweise durch eine Chlorierung, Bromierung, Fluorierung oder Mesylierung, erhalten wurden. Dabei kann das Intermediat - die Verbindung der allgemeinen Formel (2) - unmittelbar so verwendet werden, wie sie in Verfahrensstufe (2) erhalten wird.

Wenn im Rahmen der vorliegenden Erfindung vorgesehen ist, dass die Verbindung der allgemeinen Formel (2) nicht isoliert wird, so kann die Einführung der Abgangsgruppe G in der Verfahrensstufe (2) sowie die anschließende Ringschlussreaktion der Verfahrensstufe (1) als so genannte Eintopf-Reaktion durchgeführt werden.

In diesem Fall und somit in einer zweiten Ausgestaltung des Verfahrensschrittes (1) ist es möglich, den Verfahrensschritt (1) als Eintopf-Reaktion zusammen mit Verfahrensschritt (2) auszubilden. In dieser Ausgestaltung ist es möglich, auf den Basenzusatz im Verfahrensschritt (1) zu verzichten, wodurch jedoch die Gesamtausbeute über beide Verfahrensstufen etwas erniedrigt wird.

Das Produkt des Verfahrensschrittes (1) kann mittels dem Fachmann bekannten Verfahrensoperationen aufgereinigt werden, wie beispielsweise Kristallisation oder Chromatographie, wobei jedoch die Reinheit des Rohprodukts bereits ausreichend ist, um in nachfolgenden Reaktionen eingesetzt zu werden.

### Verfahrensschritt (2):

Der Verfahrensschritt (2) umfasst die Transformation der Hydroxylfunktion der Verbindung der allgemeinen Formel (3) gemäß folgender Reaktionsgleichung in eine Abgangsgruppe, ausgewählt aus der Gruppe, bestehend aus Fluor, Chlor, Brom, Iod, -OSO₂-CH₃, -O-SO₂CF₃, -O-SO₂-Ph und -O-SO₂-C₆H₄-Me:

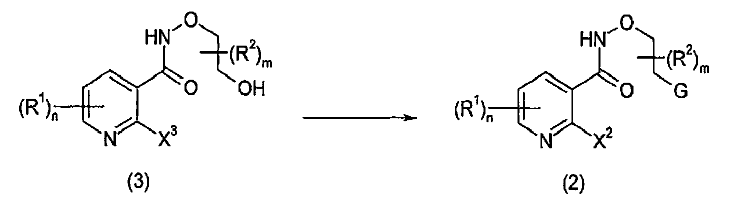

Hinsichtlich der einzelnen Reste R¹, R², X² und X³ sowie den Indizes m und n wird auf die entsprechenden Definitionen verwiesen.

Die in diesem Verfahrensschritt (2) erhaltenen Verbindungen der allgemeinen Formel (2) können als Edukt in dem Verfahrensschritt (1) des erfindungsgemäßen Verfahrens eingesetzt werden, wobei es möglich ist, die Verbindung der allgemeinen Formel (2) nach Verfahrensschritt (2) aufzuarbeiten, d.h. in isolierter und gegebenenfalls gereinigter Form zu verwenden, oder aber in nicht aufgereinigter Form einzusetzen (so genannte Eintopf-Reaktion).

Es bestehen in Abhängigkeit der Wahl der Abgangsgruppe mehrere Möglichkeiten, die Transformation gemäß Verfahrensschritt (2) durchzuführen.

Falls die Abgangsgruppe Chlor darstellt, was im Rahmen der vorliegenden Erfindung auch bevorzugt ist, können zur Chlorierung der Verbindung der allgemeinen Formel (3) beliebige Chlorierungsmittel eingesetzt werden. In Frage kommen beispielsweise Thionylchlorid (SOCl₂), Phosphorylchlorid (POCl₃), Phosgen, Diphosgen und Oxalylchlorid ((COCl)₂). Hiervon besonders bevorzugt sind Thionylchlorid (SOCl₂), Phosgen und Oxalylchlorid ((COCl)₂).

Die Menge an verwendetem Chlorierungsmittel kann in weiten Bereichen variieren. Beispielsweise beträgt die für den Verfahrensschritt (2) verwendete Menge an Chlorierungsmittel 0,8 bis 3 Moläquivalente, besonders bevorzugt 1 bis 2,5 Moläquivalente, insbesondere 1,1 bis 1,8 Moläquivalente, jeweils bezogen auf die Menge Verbindungen der allgemeinen Formel (3).

Falls die Abgangsgruppe Brom darstellt, können zur Bromierung der Verbindung der allgemeinen Formel (3) beliebige Bromierungsmittel eingesetzt werden. In Frage kommen beispielsweise Phosphortribromid (PBr₃) oder Phosphorylbromid (POBr₃). Die Menge an verwendetem Bromierungsmittel kann in weiten Bereichen variieren. Beispielsweise beträgt die für den Verfahrensschritt (2) verwendete Menge an Bromierungemittel 0,8 bis 3 Moläquivalente, besonders bevorzugt 1 bis 2,5 Moläquivalente, insbesondere 1,1 bis 1,8 Moläquivalente, jeweils bezogen auf die Menge Verbindungen der allgemeinen Formel (3).

Falls die Abgangsgruppe Fluor darstellt, können zur Fluorierung der Verbindung der allgemeinen Formel (3) beliebige Fluorierungsmittel eingesetzt werden. In Frage kommen beispielsweise (CH₃)₂NSF₃ (DAST), Deoxofluor^{®}, Yarovenko- oder Ishikava Reagenz (ClCFH-CF₂-N(C₂H₅)₂).

Die Menge an verwendetem Fluorierungsmittel kann in weiten Bereichen variieren. Beispielsweise beträgt die für den Verfahrensschritt (2) verwendete Menge an Fluorierungsmittel 0,8 bis 3 Moläquivalente, besonders bevorzugt 1 bis 1,5 Moläquivalente, insbesondere 1 bis 1,3 Moläquivalente, jeweils bezogen auf die Menge Verbindungen der allgemeinen Formel (3).

Falls die Abgangsgruppe Iod darstellt, können zur lodierung der Verbindung der allgemeinen Formel (3) beliebige Iodierungsmittel eingesetzt werden. In Frage kommen beispielsweise I₂/P oder CH₃SO₂Cl/Kl.

Die Menge an verwendetem Iodierungsmittel kann in weiten Bereichen variieren. Beispielsweise beträgt die für den Verfahrensschritt (2) verwendete Menge an lodierungemittel 0,8 bis 2 Moläquivalente, besonders bevorzugt 1 bis 1,5 Moläquivalente, insbesondere 1 bis 1,2 Moläquivalente, jeweils bezogen auf die Menge Verbindungen der allgemeinen Formel (3).

Falls die Abgangsgruppe -O-SO₂-CH₃, -OSO₂-Ph, -OSO₂-C₆H₄-CH₃ darstellt, kann zur Einführung der Abgangsgruppe in die Verbindung der allgemeinen Formel (3) Methansulfonylchlorid (CH₃-SO₂-Cl), Phenylsulfochlorid (PhSO₂Cl) oder Tolylsulfochlorid CH₃-C₆H₄SO₂-Cl verwendet werden.

Die Menge an verwendetem Methansulfonylchlorid (CH₃-SO₂-Cl), Phenylsulfochlorid (PhSO₂Cl) oder Tolylsulfochlorid (CH₃-C₆H₄SO₂Cl) kann in weiten Bereichen variieren. Beispielsweise beträgt die für den Verfahrensschritt (2) verwendete Menge an Reagenzien 0,8 bis 3 Moläquivalente, besonders bevorzugt 1 bis 2,5 Moläquivalente, insbesondere 1 bis 1,5 Moläquivalente, jeweils bezogen auf die Menge Verbindungen der allgemeinen Formel (3).

Falls die Abgangsgruppe -O-SO₂-CF₃ darstellt, kann zur Einführung der Abgangsgruppe in die Verbindung der allgemeinen Formel (3) Trifluormethylsulfonsäureanhydrid (CF₃-SO₂)₂O verwendet werden.

Die Menge an verwendetem Trifluormethylsulfonsäureanhydrid kann in weiten Bereichen variieren. Beispielsweise beträgt die für den Verfahrensschritt (2) verwendete Menge an Trifluormethylsulfonsäureanhydrid 0,8 bis 2,5 Moläquivalente, besonders bevorzugt 1 bis 2 Moläquivalente, insbesondere 1 bis 1,5 Moläquivalente, jeweils bezogen auf die Menge Verbindungen der allgemeinen Formel (3).

Der Verfahrensschritt (2) wird im Allgemeinen in Gegenwart eines Lösemittels durchgeführt. Als Lösemittel können beispielsweise organische Lösemittel verwendet werden. Beispiele von organischen Lösungsmitteln sind aromatische oder aliphatische Lösungsmittel wie Benzol, Toluol, Xylol, Mesytilen, Hexan, Heptan, Octan, Cyclohexan; aliphatische und aromatische Halogenwasserstoffe, wie Methylenchlorid, Dichlorethan, Chloroform, Kohlenstofftetrachlorid, Chlorbenzol, Dichlorbenzol; Säureamidderivate wie N,N-Dimethylformamid, N,N-Dimethylacetamid und N-Methyl-2-pyrollidon; sowie auch Carbonsäureester wie Ethylacetat; oder aber auch Dioxan, Diglym, Dimethylglycol oder THF; Nitrile wie Methylnitril, Butylnitril oder Phenylnitril. Besonders bevorzugt sind Toluol, Xylol, Dichlorbenzol, Chlorbenzol oder Ethylacetat. Hiervon sind die folgenden Lösemittel besonders bevorzugt: Methylenchlorid, Dichlorethan, Ethylacetat, N,N-Dimethylformamid, N,N-Dimethylacetamid und N-Methyl-2-pyrollidon.

Die Reaktionstemperatur, bei welcher die Umsetzung gemäß Verfahrensschritt (2) durchgeführt werden kann, kann in weiten Bereichen variieren. Beispielsweise kann die Ringschlussreaktion bei einer Temperatur von 10 bis 100 °C, vorzugsweise 20 bis 80 °C, durchgeführt werden. Die Reaktionstemperatur hängt dabei von der Reaktivität der einzelnen Verbindungen ab.

Der Verfahrensschritt (2) des erfindungsgemäßen Verfahrens wird im Allgemeinen unter Normaldruck durchgeführt. Es ist jedoch auch möglich, unter erhöhtem Druck oder vermindertem Druck - im Allgemeinen zwischen 0,1 bar und 10 bar - zu arbeiten.

Das Produkt des Verfahrensschrittes (2) kann mittels dem Fachmann bekannten Verfahrensoperationen aufgereinigt werden, wie beispielsweise Kristallisation oder Chromatographie, wobei jedoch die Reinheit des Rohprodukts bereits ausreichend ist, um in der nachfolgenden Reaktion des Verfahrensschrittes (1) eingesetzt zu werden.

Das erfindungsgemäße Verfahren liefert die gewünschten Dioxazinderivate in hoher Ausbeute und Reinheit. Das erfindungsgemäße Verfahren kann einfach und insbesondere ohne Verwendung umweltschädigender Reagenzien durchgeführt werden. Aufgrund der Möglichkeit einer Eintopf-Reaktion ist das Verfahren kostengünstig; auf entsprechende Aufarbeitungen des Intermediats und der Zielverbindung kann verzichtet werden.

Darüber hinaus sind die Verbindungen der allgemeinen Formel (2) mit G ausgewählt aus der Gruppe, bestehend aus Fluor, Chlor, Brom, Iod, -OSO₂-CH₃, -O-SO₂CF₃, -O-SO₂-Ph und -O-SO₂-C₆H₄-Me neu.

Weiterer Gegenstand der vorliegenden Erfindung sind somit Verbindungen der allgemeinen Formel (2) 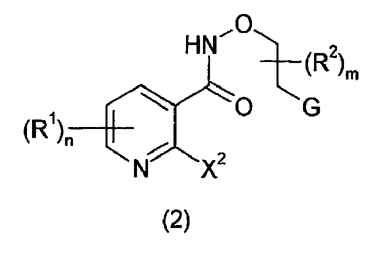 in welchen die einzelnen Substituenten die folgende Bedeutung aufweisen:
- G: Fluor, Chlor, Brom, Iod, -OSO₂-CH₃, -O-SO₂CF₃, -O-SO₂-Ph und -O-SO₂-C₆H₄-Me;
- X²: Fluor, Chlor, Brom, Iod, SCN, oder S-R³, wobei R³ für Wasserstoff; gegebenenfalls substituiertes C₁-C₆-Alkyl; gegebenenfalls substituiertes C₃-C₆-Cycoalkyl; -(CH₂)ᵣ-C₆H₅ mit r = 0 bis 6, wobei der Alkylrest -(CH₂)ᵣ- gegebenenfalls substituiert sein kann; oder für den Rest 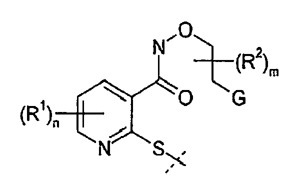 steht,
- R¹: Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl-und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
- n: eine ganzzahlige Zahl von 0 bis 2;
- R²: unabhängig voneinander jeweils gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, wobei die Substituenten unabhängig voneinander ausgewählt sein können aus Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, (C₁-C₆-Alkoxy)carbonyl, (C₁-C₆-Alkyl)carbonyl oder C₃-C₆-Trialkylsilyl; und m eine ganzzahlige Zahl von 0 bis 4.

In einer bevorzugten Ausführungsform weisen die Substituenten folgende Bedeutung auf:
- G: Fluor, Chlor, Brom und Iod.
- X²: Chlor, S-R³, wobei
R³ für gegebenenfalls substituiertes C₁-C₆-Alkyl; gegebenenfalls substituiertes C₃-C₆-Cycoalkyl; -(CH₂)ᵣC₆H₅ mit r = 0 bis 4 steht, wobei der Alkylrest -(CH₂)ᵣ- gegebenenfalls substituiert sein kann;
- R¹: Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
- n: 0 oder 1;
- R²: jeweils unabhängig voneinander für gegebenenfalls einfach oder mehrfach, gleich oder verschieden substituiertes C₁-C₄-Alkyl, C₃-C₆-Cycloalkyl, wobei die Substituenten unabhängig voneinander ausgewählt sein können aus Halogen, Cyano, Nitro, Hydroxy, C₁-C₄-Alkoxy, C₁-C₄-Haloalkoxy, C₁-C₄-Alkylthio, C₁-C₄-Alkylsulfinyl und C₁-C₄-Alkylsulfonyl; und
- m: eine ganzzahlige Zahl von 0 bis 2.

Darüber hinaus sind folgende Bedeutungen der Substituenten der Verbindungen der allgemeinen Formeln (2) besonders bevorzugt:
- G: Chlor;
- X²: Chlor, S-R³, wobei
R³ für gegebenenfalls substituiertes C₁-C₆-Alkyl; gegebenenfalls substituiertes C₃-C₆-Cycoalkyl; -(CH₂)ᵣ-C₆H₅ mit m = 0 bis 2 steht, wobei der Alkylrest -(CH₂)ᵣ- gegebenenfalls substituiert sein kann;
- R¹: Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
- n: 0 oder 1;
- R²: gegebenenfalls einfach oder mehrfach, gleich oder verschieden substituiertes C₁-C₄-Alkyl, wobei die Substituenten unabhängig voneinander ausgewählt sein können aus Halogen, Cyano, Nitro, C₁-C₄-Alkoxy, C₁-C₄-Haloalkoxy; und
- m: 0 oder 1.

Darüber hinaus sind folgende Bedeutungen der Substituenten der Verbindungen der allgemeinen Formel (2) insbesondere bevorzugt:
- G: Chlor;
- X: S-CH₂-C₆H₅;
- n: 0;
- m: 0.

Diese Verbindungen können gemäß den oben beschrieben Verfahren erhalten werden.

Die Erfindung wird nunmehr anhand des nachfolgenden Ausführungsbeispiels näher erläutert werden, ohne sie jedoch auf dieses zu beschränken.

### Ausführungsbeispiel:

### Beispiel 1:

### Herstellung von 2-(Benzylthio)-N-(2-chloroethoxy)nicotinamid

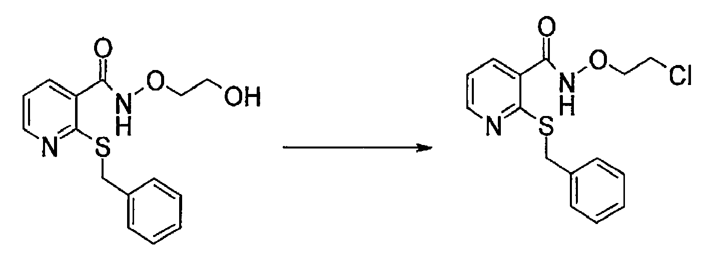

11,4 g (2-(Benzylthio)-N-(2-hydroxyethoxy)nicotinamid wurden in 50 ml N,N-Dichlormethan vorgelegt und 6.6 g Thionylchlorid bei 30 °C zugetropft. Das Reaktionsgemisch wurde 1 Stunde bei Raumtemperatur nachgerührt und dann wurde das Gemisch mit 100 ml Wasser verdünnt. Die Suspension wurde 1 Stunde bei 20 °C nachgerührt und der Niederschlag wurde abgesaugt und mit Wasser gewaschen.

Man erhielt 11,47 g, 95 % d. T. des Produktes (Smp. 122-124 °C).

¹H NMR (DMSO-d₆): 3.81 (m, 2H), 4.15 (m, 2H), 4.4 (s, 2H), 7.2-7.4 (m, 6H), 7.8 (dd, 1 H), 8.5 (dd, 1 H).

### Beispiel 2:

### Herstellung von 3-[2-(Benzylthio)pyridin-3-yl]-5,6-dihydro-1,4,2-dioxazin (ohne Base)

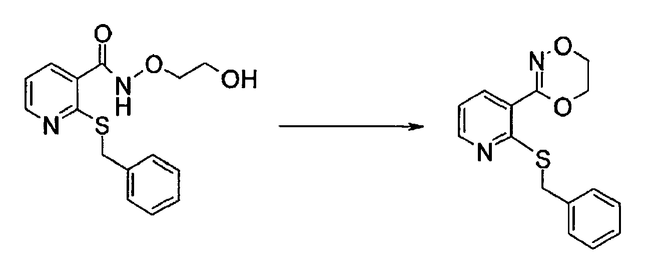

114 g (2-(Benzylthio)-N-(2-hydroxyethoxy)nicotinamid wurden in 500 ml THF vorgelegt und 65.8 g Thionylchlorid wurden bei 10 °C zugetropft. Das Reaktionsgemisch wurde 1 Stunde bei Raumtemperatur nachgerührt und dann wurde der Niederschlag (HCI-Salz des Produktes) abfiltriert und mit Wasser und NaHCO₃-Lösung gewaschen und getrocknet.

Man erhielt ca. 65 g des Produktes mit Smp. 63 - 65 °C.

¹H NMR (DMSO-d₆): 4.2 (d, 2H), 4.4 (s, 2H), 4.45 (d, 2H), 7.2-7.4 (m, 6H), 7.7 (d, 1H), 8.5 (d, 1H).

### Beispiel 3:

### Herstellung von 3-[2-(Benzylthio)pyridin-3-yl]-5,6-dihydro-1,4,2-dioxazin (mit Base)

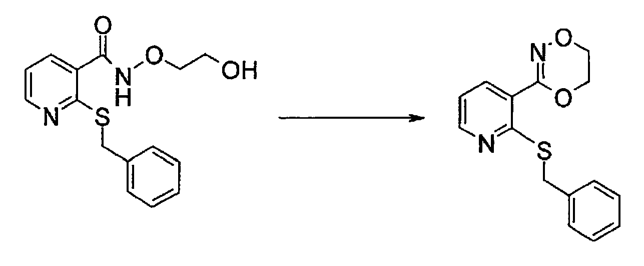

114 g (2-(Benzylthio)-N-(2-hydroxyethoxy)nicotinamid wurden in 500 ml N,N-Dimethylacetamid vorgelegt und 65.8 g Thionylchlorid zugetropft. Das Reaktionsgemisch wurde 1 Stunde bei Raumtemperatur nachgerührt und dann wurde 306 g Pottasche-Lösung bei Raumtemperatur zugetropft. Das Gemisch wurde auf 70 °C aufgeheizt und ca. 1 Stunde bei dieser Temperatur nachgerührt. Wasser wurde zugegeben und die Suspension wurde 3 Stunden bei 20 °C nachgerührt. Der Niederschlag wurde abgesaugt und mit Wasser gewaschen.

Man erhält 103 g, 95 % d. T. (Reinheit 98 %, 100 % LC), Smp. 62 - 64 °C.

## Patentansprüche

1. Verfahren zur Herstellung von Dioxazin-Derivaten der Formel (1) 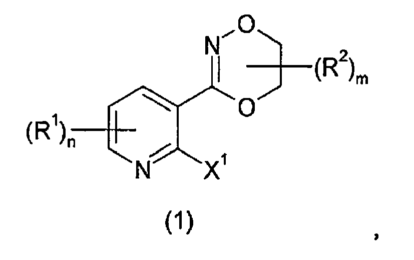 worin die einzelnen Substituenten die folgende Bedeutung aufweisen:
X¹ Fluor, Chlor, Brom, Iod, SCN, oder S-R³, wobei
R³ für Wasserstoff; gegebenenfalls substituiertes C₁-C₆-Alkyl; gegebenenfalls substituiertes C₃-C₆-Cycoalkyl; -(CH₂)ᵣ-C₆H₅ mit r = 0 bis 6, wobei der Alkylrest -(CH₂)ᵣ-gegebenenfalls substituiert sein kann; oder 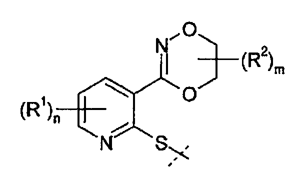
wobei die Substituenten R¹ und R² und die Indizes m und n dieselbe Bedeutung wie in der allgemeinen Formel (1) aufweisen; steht;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n eine ganzzahlige Zahl von 0 bis 2;
R² unabhängig voneinander jeweils gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, wobei die Substituenten unabhängig voneinander ausgewählt sein können aus Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, (C₁-C₆-Alkoxy)carbonyl, (C₁-C₆-Alkyl)carbonyl oder C₃-C₆-Trialkylsilyl; und m eine ganzzahlige Zahl von 0 bis 4,
**dadurch gekennzeichnet, dass**
die Dioxazin-Derivate der allgemeinen Formel (1) durch einen Ringschluss ausgehend von einer Verbindung der allgemeinen Formel (2) hergestellt werden (Verfahrensschritt (1)): 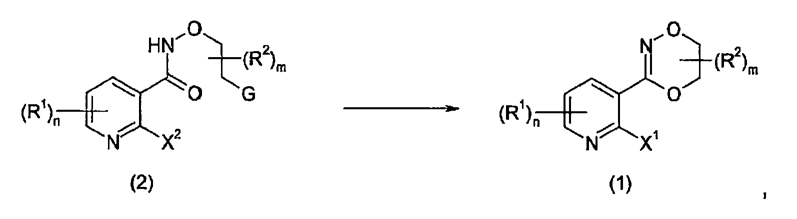 wobei die Substituenten R¹, R² und X¹ sowie die Indizes n und m wie oben stehend definiert sind; G eine Abgangsgruppe darstellt, welche ausgewählt ist aus der Gruppe, bestehend aus Fluor, Chlor, Brom, Iod, -OSO₂-CH₃, -O-SO₂CF₃, -O-SO₂-Ph und -O-SO₂-C₆H₄-Me, und
X² für Fluor, Chlor, Brom, Iod, SCN, oder S-R³ steht, wobei
R³ für Wasserstoff; gegebenenfalls substituiertes C₁-C₆-Alkyl; gegebenenfalls substituiertes C₃-C₆-Cycoalkyl; -(CH₂)ᵣ-C₆H₅ mit r = 0 bis 6, wobei der Alkylrest -(CH₂)ᵣ- gegebenenfalls substituiert sein kann; oder für den Rest 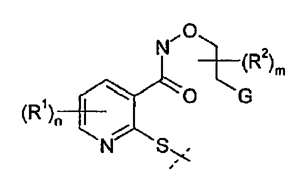
steht, wobei die Substituenten R¹ und R² und die Indizes m und n in dem Rest dieselbe Bedeutung wie in der allgemeinen Formel (1) aufweisen.

2. Verfahren nach Anspruch 1, **dadurch gekennzeichnet, dass** die Verbindung der allgemeinen Formel (2) erhältlich ist durch Transformation der Hydroxylfunktion der Verbindung der allgemeinen Formel (3) in eine Abgangsgruppe, ausgewählt aus der Gruppe, bestehend aus Fluor, Chlor, Brom, Iod, -OSO₂-CH₃, -O-SO₂CF₃, -O-SO₂-Ph und -O-SO₂-C₆H₄-Me: 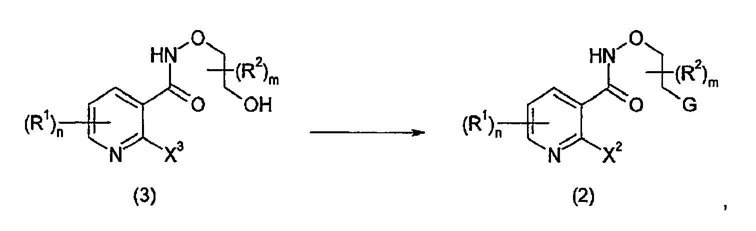 wobei X³ für Fluor, Chlor, Brom, Iod, SCN, oder S-R³ steht, wobei R³ für Wasserstoff; gegebenenfalls substituiertes C₁-C₆-Alkyl; gegebenenfalls substituiertes C₃-C₆-Cycloalkyl; -(CH₂)ᵣ-C₆H₅ mit r = 0 bis 6, wobei der Alkylrest -(CH₂)ᵣ- gegebenenfalls substituiert sein kann; oder für den Rest 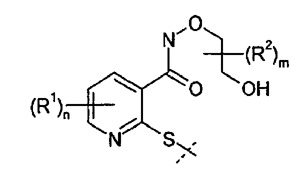 steht, wobei die Substituenten R¹ und R² und die Indizes m und n in dem Rest dieselbe Bedeutung wie in der allgemeinen Formel (1) aufweisen.

3. Verfahren nach Anspruch 1 oder 2, **dadurch gekennzeichnet, dass** die Cyclisierung in Verfahrensschritt (1) in Gegenwart einer Base durchgeführt wird.

4. Verfahren nach einem der Ansprüche 1 bis 3, **dadurch gekennzeichnet, dass** die Umsetzung gemäß Verfahrensschritt (1) bei einer Temperatur von 20 bis 100 °C durchgeführt wird.

5. Verfahren nach Anspruch 2 oder 3, **dadurch gekennzeichnet, dass** die Abgangsgruppe Chlor darstellt und in Verfahrensschritt (2) eine Chlorierung mittels einem Chlorierungsmittel durchgeführt wird, welches ausgewählt ist aus der Gruppe, bestehend aus Thionylchlorid (SOCl₂), Phosphorylclorid (POCl₃), Phosgen, Diphosgen und Oxalylchlorid ((COCl)₂).

6. Verfahren nach einem der Ansprüche 2 bis 5, **dadurch gekennzeichnet, dass** die Umsetzung gemäß Verfahrensschritt (2) bei einer Temperatur von 10 bis 100 °C durchgeführt wird.

7. Verfahren nach einem der Ansprüche 2 bis 6, **dadurch gekennzeichnet, dass** die Verfahrensschritte (1) und (2) als Eintopf-Reaktion ohne Isolierung der Verbindung der allgemeinen Formel (2) durchgeführt wird.

8. Verfahren nach Anspruch 7, **dadurch gekennzeichnet, dass** die Eintopf Reaktion ohne Zugabe einer Base durchgeführt wird.

9. Verbindungen der allgemeinen Formel (2) 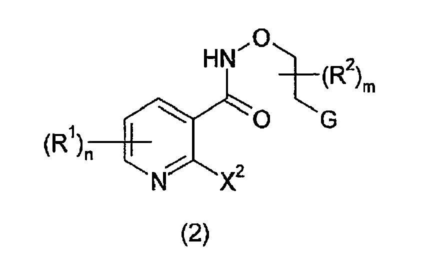 in welchen die einzelnen Substituenten die folgende Bedeutung aufweisen:
G Fluor, Chlor, Brom, Iod, -OSO₂-CH₃, -O-SO₂CF₃, -O-SO₂-Ph und -O-SO₂-C₆H₄-Me;
X² Fluor, Chlor, Brom, Iod, SCN, oder S-R³, wobei
R³ für Wasserstoff; gegebenenfalls substituiertes C₁-C₆-Alkyl; gegebenenfalls substituiertes C₃-C₆-Cycoalkyl; -(CH₂)ᵣ-C₆H₅ mit r = 0 bis 6, wobei der Alkylrest -(CH₂)ᵣ-gegebenenfalls substituiert sein kann; oder für den Rest 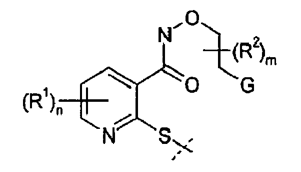
steht, wobei die Substituenten R¹ und R² und die Indizes m und n in dem Rest dieselbe Bedeutung wie in der allgemeinen Formel (1) aufweisen;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n eine ganzzahlige Zahl von 0 bis 2;
R² unabhängig voneinander jeweils gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, wobei die Substituenten unabhängig voneinander ausgewählt sein können aus Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, (C₁-C₆-Alkoxy)carbonyl, (C₁-C₆-Alkyl)carbonyl oder C₃-C₆-Trialkylsilyl; und m eine ganzzahlige Zahl von 0 bis 4.

10. Verbindung der allgemeinen Formel (2) nach Anspruch 9, wobei die Substituenten die folgende Bedeutung aufweisen:
G Fluor, Chlor, Brom und Iod.
X² S-R³, wobei
R³ für gegebenenfalls substituiertes C₁-C₆-Alkyl; gegebenenfalls substituiertes C₃-C₆-Cycoalkyl; -(CH₂)ᵣ-C₆H₅ mit r = 0 bis 4 steht, wobei der Alkylrest -(CH₂)ᵣ- gegebenenfalls substituiert sein kann;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkyls ulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n 0 oder 1;
R² jeweils unabhängig voneinander für gegebenenfalls einfach oder mehrfach, gleich oder verschieden substituiertes C₁-C₄-Alkyl, C₃-C₆- Cycloalkyl, wobei die Substituenten unabhängig voneinander ausgewählt sein können aus Halogen, Cyano, Nitro, Hydroxy, C₁-C₄-Alkoxy, C₁-C₄-Haloalkoxy, C₁-C₄-Alkylthio, C₁-C₄-Alkylsulfinyl und C₁-C₄-Alkylsulfonyl; und
m eine ganzzahlige Zahl von 0 bis 2.

11. Verbindung der allgemeinen Formel (2) nach Anspruch 9 oder 10, wobei die Substituenten die folgende Bedeutung aufweisen:
G Chlor;
X² S-R³, wobei
R³ für gegebenenfalls substituiertes C₁-C₆-Alkyl; gegebenenfalls substituiertes C₃-C₆-Cycloalkyl; -(CH₂)ᵣ-C₆H₅ mit r = 0 bis 6 steht, wobei der Alkylrest -(CH₂)ᵣ- gegebenenfalls substituiert sein kann;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n 0 oder 1;
R² gegebenenfalls einfach oder mehrfach, gleich oder verschieden substituiertes C₁-C₄-Alkyl, wobei die Substituenten unabhängig voneinander ausgewählt sein können aus Halogen, Cyano, Nitro, C₁-C₄-Alkoxy, C₁-C₄-Haloalkoxy; und
m 0 oder 1.

12. Verbindungen der allgemeinen Formel (2) nach einem der Ansprüche 9 bis 11, wobei die Substituenten die folgende Bedeutung aufweisen:
G Chlor;
X² S-CH₂C₆H₅;
n 0; und
m 0.
